Europäisches Patentamt

European Patent Office

Office européen des brevets

(19)

(11) Publication number: **0 373 417**
**A1**

## (12) EUROPEAN PATENT APPLICATION

(21) Application number: **89121954.5**

(22) Date of filing: **28.11.89**

(51) Int. Cl.5: **A61K 9/20, A61K 9/22,**
**A61K 9/26, A61K 9/30,**
**A61K 9/52**

(30) Priority: **30.11.88 US 278057**

(43) Date of publication of application:
**20.06.90 Bulletin 90/25**

(84) Designated Contracting States:
**GR**

(71) Applicant: **SCHERING CORPORATION**
**2000 Galloping Hill Road**
**Kenilworth New Jersey 07033(US)**

(72) Inventor: **Chang, Richard R.**
**1451 S.W. 88th Way**
**Pembroke Pines Florida 33025(US)**
Inventor: **Pereira-Rosario, Ronald**
**203 N.E. 1st Street**
**Dania Florida 33004(US)**
Inventor: **Rudnic, Edward M.**
**6964 South Grande Drive**
**Boca Raton Florida 33433(US)**

(74) Representative: **von Kreisler, Alek,**
**Dipl.-Chem. et al**
**Patentanwälte Von Kreisler-Selting-Werner**
**Deichmannhaus am Hauptbahnhof**
**D-5000 Köln 1(DE)**

(54) Sustained release diltiazem formulation.

(57) A sustained release diltiazem tablet is disclosed which exhibits a unique dissolution profile due in large measure to the inclusion of the drug into a hydrophobic matrix. In particular, the sustained release diltiazem formulation disclosed herein is suitable for once-a-day administration.

EP 0 373 417 A1

## SUSTAINED RELEASE DILTIAZEM FORMULATION

### SUMMARY

The present invention encompasses sustained release oral dosage forms and formulations for medicinal agents, and in particular for diltiazem.

One such oral dosage form is a sustained release tablet, comprising an effective amount of active ingredient and excipients which may be compressed into a suitable oral dosage form, and which may be coated with one or more coating agents. The tablet coating may optionally contain diltiazem for immediate release.

In a particular formulation described herein, a sustained release tablet may contain diltiazem or a pharmaceutically acceptable salt thereof in combination with excipients such as glyceryl monostearate, sucrose, microcrystalline cellulose and povidone.

The matrix formed by tablet compression is hydrophobic in nature.

### BACKGROUND OF THE INVENTION

Numerous references disclose diltiazem in sustained release formulations which utilize microencapsulation technology. Examples are the following:

U.S. Patent no. 4,452,042, issued to Samejima et al. on September 17, 1985;

U.S. Patent no. 4,462,982, issued to Samejima et al. on July 31, 1984;

U.S. Patent no. 4,443,497 issued to Samejima et al. on April 17, 1984; and

U.S. Patent 4,411,933, issued to Samejima et al. on October 25, 1983.

Similarly, numerous publications have disclosed devices which rely upon an osmotically regulated membrane for the controlled delivery of pharmaceuticals, such as diltiazem. For example are the following:

Belgian Application 900817, published on February 1, 1985 discloses a device comprising a semipermeable wall, an osmopolymer, such as poly(ethylene oxide) and an active ingredient.

Belgian Appl. No. 900,824 also published on February 1, 1985 discloses a core and a membrane having variable permeability;

Belgian Appl. No. 898,819, published on May 30, 1984, discloses a device for controlled drug delivery containing two compositions, including poly(ethyleneoxide);

Belgian Appl. No. 903,540 published February 17, 1986 discloses a sustained release powder, which can be formulated into an ointment, suspension etc.

Belgian Appl. No. 901,359 published April 16, 1985 discloses a controlled release diltiazem formulation containing granules and a semipermeable external membrane.

Japanese Appl. No. 175,144 published on April 13, 1984, discloses a sustained release thermoset or thermoplastic resin;

Japanese Appl. No. 170,440 published on April 5, 1984, discloses a sustained release tablet which utilizes hardened oil;

Japanese Kokai 62/5915, published January 12, 1987, discloses diltiazem in combination with an acrylic acid resin;

Japanese Kokai 61/212517, published September 20, 1986 discloses the use of diltiazem in combination with hydrogenated oils;

Japanese Kokai 59/10512 published January 20, 1984, discloses diltiazem microencapsulated in ethylcellulose;

Panoz and Geohagan, U.S. Patent 4,721,619 discloses an alternating arrangement (between 50 and 200 layers) of diltiazem, organic acid and lubricant layers and polymeric material layers built upon a central inert core.

However, none of the references disclose a sustained release diltiazem tablet formulation utilizing a uniformly dispersed hydrophobic matrix.

### DETAILED DESCRIPTION

The present invention relates to a novel sustained release tablet, useful in that it exhibits unexpectedly prolonged activity, a uniform dissolution rate, and formulation stability over an extended period of time. The sustained release diltiazem tablets described herein will be suitable for once a day and twice daily administration.

The tablets of the invention utilize a hydrophobic matrix, into which the active ingredient is incorporated. As used herein, the term "hydrophobic matrix" refers to the nature of the major pharmaceutically acceptable excipients into which the active ingredient such as diltiazem is incorporated prior to tableting. The components of the hydrophobic matrix are generally recognized as non-therapeutic, and are useful to impart the required dissolution characteristics to the sustained release tablets.

"Excipients" as used herein refers to non-therapeutic ingredients which may be incorporated into the formulation. Hence, the components used to create the hydrophobic matrix are referred to as excipients. Other formulation excipients used in the manufacture of the sustained release diltiazem tablets include diluents, binders, fillers, glidants, lubricants, disintegrants, and other pharmaceutically acceptable non-therapeutic agents.

A preferred sustained release tablet falling within the scope of the invention utilizes diltiazem hydrochloride or a solvate thereof as the active ingredient. Preferably the amount of the active ingredient, such as diltiazem, will be present at about 20 to 500 mg per tablet, more preferably at about 30 to 360 mg per tablet and most preferably at about 50 to 250 mg per tablet, representing about 10 to about 40 percent, preferably about 10 to about 20 percent of the total tablet weight.

The diltiazem utilized herein also encompasses other pharmaceutically acceptable acid addition salt forms thereof, as well as other pharmaceutically acceptable salts and esters thereof. As described above, the diltiazem used in Examples 1 to 7 below is the hydrochloride salt. Also included herein are stereospecific salt forms of diltiazem, both in pure form and racemic mixture. One such example is the (d,l) lactate form of diltiazem.

The hydrophobic matrix utilized herein may be comprised largely of a mixture of mono- and diglycerides, e.g., glyceryl monostearate, and one or more of other known water insoluble excipients such as ethyl cellulose, cellulose acetate, calcium phosphate, cellulose acetate butyrate and microcrystalline cellulose. The hydrophobic matrix formed is generally non-water soluble, and serves to reduce the rate of dissolution.

The preferred hydrophobic matrix containing glyceryl monostearate (Atmul 84S, U.S. Emulsifier, Paris, IL) and microcrystalline cellulose (Avicel, FMC Corporation) can contain relative portions of the two ingredients ranging from about 2:4 to about 4:2. The hydrophobic matrix therefore may form from about 40 to about 90 percent of the total tablet weight.

Sugar, and in particular confectioner's sugar (size) 6 X can be used in the formulation described herein as a diluent. By increasing the concentration of sugar, an increased rate of dissolution will result. The diluent contained in the formulation can comprise from 0 to about 20 percent, being inversely related to the amount of hydrophobic matrix material present. For example, if a particularly long acting sustained release diltiazem tablet is desired, a larger amount of hydrophobic matrix material and a correspondingly low concentration of sugar is utilized.

The tablet can utilize a conventional binder during the granulation step. Examples of binders are povidone, ethyl cellulose, acacia and a sugar solution, with povidone being preferred. The concentration of binder ranges from 0 to about 10 percent, preferably about 3 to about 10 percent, with the more preferred concentration being 4 to 5 percent.

During preparation of the sustained release tablets, the diltiazem, hydrophobic matrix material, diluent (if present) and binder (if present) are mixed and then typically granulated. A lubricant is preferably added prior to tablet compression. Alternatively, a direct compression method, without employing a binder, may be performed. Typical lubricants are magnesium stearate, stearic acid, sodium stearyl fumarate, hydrogenated vegetable oil, calcium stearate, talcum and corn starch, with magnesium stearate being preferred. The lubricant, when present, may be added in an amount ranging from 0.5 to about 2 percent. The preferred amount of lubricant is about 1 percent of the total tablet weight.

The granulation step can be performed using a back granulation technique, wherein the diltiazem, hydrophobic matrix materials, diluent and binder are combined. By adding part of the microcrystalline cellulose at the end, less water is used to perform a wet granulation, thereby reducing drying time.

The granulation technique, using the preferred ingredients described herein, and the back granulation technique described above, may be performed by either (1) using a binder solution (wet method); or (2) using a solvent as a granulating agent and a binder in the dry form (dry method). However, the preferred granulation technique is the dry method. After granulation or mixing and after the addition of a lubricant, conventional tableting may be performed.

3

Tablet cores, i.e., the tableted hydrophobic matrix, may be film coated if desired. The film coating may be non-functional to regulate drug release or may act as a barrier to delay release of diltiazem.

Typical non-regulating coatings comprise water-soluble agents applied using organic or, preferably, aqueous solvents using conventional spray technology. Such coatings provide tablets with increased hardness, better appearance, taste-masking and protection against light and moisture. Typical water-soluble coating agents are swellable hydrophilic polymers, and are selected based upon solubility, viscosity in solution, drying time, etc. Typical swellable hydrophilic polymers include cellulosic ethers such as hydroxypropyl methylcellulose, hydroxypropylcellulose, hydroxyethylcellulose, and the sodium salt of carboxymethylcellulose, or mixtures thereof. When present, the amount of hydrophilic polymer comprises from about 0.5 to about 5% of the total tablet weight. An aqueous film coating procedure may be accomplished with or without a surfactant.

A preferred non-regulatory coating includes hydroxypropyl methylcellulose (HPMC) USP 2910, more preferably an HPMC USP 2910 having a viscosity designation E5, and most preferably those sold under the trade name Methocel E5 Premium (Dow Chemical). The amount of HPMC E5 used, when present, comprises from about 0.5 to about 2 percent of the total tablet weight. Another coating material is HPMC USP 2910, more preferably an HPMC USP 2910 having viscosity designation E50, and most preferably those sold under the trade name Methocel E50 Premium (Dow Chemical). The amount of HPMC E50 used, when present, similarly comprises from about 0.5 to 2 percent of the total tablet weight. In the viscosity designations, "E" refers to USP 2910 and the number designation refers to the viscosity in a 2% aqueous solution (i.e., E5 has a viscosity of 5 cps and E50 has a viscosity of 50 cps).

Where the tablet coating is to act as a barrier to further regulate the release of diltiazem, water insoluble polymers and enteric polymers (i.e., water-soluble at high pH) or combinations thereof can be used, again using conventional techniques. Examples of water-insoluble polymers are ethyl cellulose, cellulose acetate butyrate, cellulose propionate and copolymers of acrylic and methacrylic acid esters. Examples of enteric polymer materials are cellulose acetate phthalate, cellulose acetate trimellitate, polyvinyl acetate phthalate and acrylic resins. The amount of water insoluble polymer, enteric polymer or combination thereof used, when present, comprises from about 0.5 to about 5% of the total tablet weight.

Other coating agents, such as plasticizers, may be included in either the water soluble or barrier coating solution. Examples of plasticizers are diethyl phthalate, dibutyl phthalate, triacetin, citric acid esters and polyethylene glycol, with polyethylene glycol 3350 (wherein 3350 refers to the average molecular weight), also known as PEG 3350, being preferred. The plasticizer tends to make the film coat flexible, and may be included in an amount ranging from about 0.1 to 1 percent of the total tablet weight.

A liquid color dispersion such as a white color dispersion comprising mixture of propylene glycol, povidone and titanium dioxide may be included in the coating step to impart a uniformly colored, finished appearance to the tablets. Such a mixture, when used, may comprise from about 0.5 to about 3 percent of the total tablet weight.

Barrier films may also incorporate a water-soluble channelling agent such as HPMC or another hydrophilic polymer identified above which will dissolve and create pores to facilitate dissolution. Alternatively, a water-insoluble but water-permeable polymer such as acrylic-methacrylic copolymer may be used to facilitate passage of water. Channeling agents, when present, are present in a concentration from about 0.5 to about 2% of the total tablet weight.

In another aspect of the invention, a portion of the diltiazem dose is incorporated into a water soluble binder and coated on the outside of the film-coated tablet core to provide immediate release of a portion of the diltiazem, thereby minimizing some of the first pass effect of metabolism in the liver. The amount of diltiazem in the tablet core is from about 75% to about 95%, preferably about 90 to about 95% of the total amount of diltiazem present. Typical components and concentrations of the water-soluble binder are as described above for the water-soluble tablet coatings.

Typical tablet cores of the present invention therefore comprise the following ingredients in the following concentrations:

a) 10 to 40 percent diltiazem hydrochloride;

b) 20 to 50 percent glyceryl monostearate;

c) 0 to 20 percent confectioner's sugar;

d) 20 to 50 percent microcrystalline cellulose;

e) 0 to 10 percent povidone; and

f) 0 to 2 percent magnesium stearate. Preferred ingredients and concentrations for the tablet cores are as follows:

a) 10 to 20 percent diltiazem hydrochloride;

b) 20 to 40 percent glyceryl monostearate;

c) 0 to 20 percent confectioner's sugar;

d) 30 to 40 percent microcrystalline cellulose;

e) 4 to 5 percent povidone; and

f) 0 to 2 percent magnesium stearate. A preferred water-soluble coating is as follows:

1 to 2 percent hydroxypropyl methylcellulose E5;

1 to 2 percent hydroxypropyl methylcellulose E50;

0.1 to 1 percent polyethylene glycol; and

2 to 3 percent color dispersion.

For the diltiazem-containing coating, 1-2% diltiazem may be added to the above water-soluble coating. Percentages above are based on total final (i.e. coated) table weight.

The following are specific examples of tablet formulations falling within the scope of the invention. However, the scope of the claims is not to be limited thereby.

| EXAMPLE 1 | | |
|---|---|---|
| Ingredient | mg per tablet | % (w/w) |
| 1. diltiazem HCl | 90.0 | 13.04 |
| 2. glyceryl monostearate | 160.8 | 23.30 |
| 3. confectioner's sugar 6X (N.F.) | 115.8 | 16.78 |
| 4. microcrystalline cellulose (N.F.) | 244.2 | 35.39 |
| 5. povidone (USP K-90) | 32.2 | 4.67 |
| 6. magnesium stearate | 6.4 | 0.93 |
| 7. hydroxypropyl methylcellulose (E5) | 11.3 | 1.64 |
| 8. hydroxypropyl methylcellulose (E50) | 8.6 | 1.25 |
| 9. polyethylene glycol (NF M.W. 3350) | 4.0 | 0.58 |
| 10. color dispersion | 16.7 | 2.42 |
| TOTAL: | 690.0 | 100.0% |

Combine ingredients 1 through 4, or 1 through 5, mix and granulate, using water in a back granulation technique. Add magnesium stearate, and compress with a conventional tableting procedure.

Combine ingredients 7 to 10 using an aqueous solvent, and apply this film coating to the tablets if desired.

The formulation described above in example 1 has been tested for in vitro dissolution, both in coated and uncoated form, and it demonstrates approximately 100 percent dissolution over a 24 hour period, as demonstrated below in Table 1.

TABLE I

| DISSOLUTION DATA FOR SUSTAINED RELEASE DILTIAZEM TABLETS | | | | | | | | | | |
|---|---|---|---|---|---|---|---|---|---|---|
| | HOURS (percent dissolution) | | | | | | | | | |
| DESCRIPTION | 1 | 2 | 4 | 6 | 8 | 10 | 12 | 16 | 20 | 24 |
| Tablet of EXAMPLE 1 (Uncoated) | 30.5 | 42 | 63 | 77 | 85 | 89 | 90 | ND | ND | 95 |
| 29 | 45 | 68 | ND | 88 | ND | 92 | 97 | 97 | 98 | |
| 11 | 23 | 39 | 49 | 60 | 72 | 80 | ND | ND | 93 | |
| 31 | 44 | 61 | ND | 83 | ND | 92 | 96 | 97 | 98 | |
| Tablet of EXAMPLE 1 (Coated) | 28 | 43 | 63 | 79 | 87 | 90 | 92 | ND | ND | 96 |
| 25 | 43 | 65 | 82 | 89 | 92 | 95 | ND | ND | 101 | |
| 28 | 46 | 70 | ND | 93 | ND | 98 | 101 | 102 | 103 | |
| 20 | 28 | 42 | 54 | 63 | 72 | 81 | ND | ND | 95 | |
| 17 | 27 | 41 | 53 | 62 | 71 | 80 | ND | ND | 92 | |
| ND = NOT DETERMINED | | | | | | | | | | |

Examples 2 through 4 are further examples of pharmaceutical compositions that have been made by the methods described in Example 1.

| EXAMPLE 2 | | |
|---|---|---|
| Ingredient | mg/tablet | % (w/w) |
| 1. diltiazem HCl | 90.0 | 13.04 |
| 2. glyceryl monostearate | 276.6 | 40.08 |
| 3. confectioner's sugar 6X (NF) | 0 | 0 |
| 4. microcrystalline cellulose (NF) | 244.2 | 35.39 |
| 5. povidone (USP K-90) | 32.2 | 4.67 |
| 6. magnesium stearate | 6.4 | 0.93 |
| 7. hydroxypropyl methylcellulose (E5) | 11.3 | 1.64 |
| 8. hydroxypropyl methylcellulose (E50) | 8.6 | 1.25 |
| 9. polyethylene glycol (NF M.W. 3350) | 4.0 | 0.58 |
| 10. color dispersion | 16.7 | 2.42 |
| Total: | 690.0 | 100.0% |

| EXAMPLE 3 | | |
|---|---|---|
| Ingredient | mg/tablet | % (w/w) |
| 1. diltiazem HCl | 90.0 | 18.69 |
| 2. glyceryl monostearate | 122.8 | 25.51 |
| 3. confectioner's sugar 6x (NF) | 30.1 | 6.26 |
| 4. microcrystalline cellulose (NF) | 185.8 | 38.60 |
| 5. povidone (USP K-90) | 21.1 | 4.39 |
| 6. magnesium stearate | 3.2 | 0.66 |
| 7. hydroxypropyl methylcellulose (E5) | 7.9 | 1.64 |
| 8. hydroxypropyl methylcellulose (E50) | 6.0 | 1.25 |
| 9. polyethylene Glycol (NF M.W. 3350) | 2.8 | 0.58 |
| 10. color dispersion | 11.7 | 2.42 |
| Total: | 481.4 | 100.0 |

| EXAMPLE 4 | | |
|---|---|---|
| Ingredient | mg/tablet | % (w/w) |
| 1. diltiazem HCl | 90.0 | 18.69 |
| 2. glyceryl monostearate | 155.6 | 32.33 |
| 3. confectioner's sugar 6x (NF) | 0 | 0 |
| 4. microcrystalline Cellulose (NF) | 183.1 | 38.04 |
| 5. povidone (USP) | 21.1 | 4.39 |
| 6. magnesium Stearate | 3.2 | 0.66 |
| 7. hydroxypropyl methylcellulose (E5) | 7.9 | 1.64 |
| 8. hydroxypropyl methylcellulose (E50) | 6.0 | 1.25 |
| 9. polyethylene glycol (NF M.W. 3350) | 2.8 | 0.58 |
| 10. color dispersion (Solids) | 11.7 | 2.42 |
| Total | 481.4 | 100.0 |

Pharmaceutical compositions as described in Examples 1-4 were also made which contained diltiazem in the amount of 120, 180 and 240 mg per tablet.

| EXAMPLE 5 | | |
|---|---|---|
| Ingredient | mg/tablet | % (w/w) |
| 1. diltiazem HCl | 180.0 | 19.45 |
| 2. glyceryl monostearate | 311.6 | 33.68 |
| 3. microcrystalline cellulose, NF | 366.6 | 39.62 |
| 4. povidone USP | 42.2 | 4.56 |
| 5. magnesium stearate | 6.4 | 0.69 |
| 6. cellulose acetate phthalate | 14.8 | 1.60 |
| 7. diethyl phthalate | 3.7 | 0.40 |
| Total: | 925.3 | 100.00 |

Prepare tablet cores from ingredients 1 through 5 as described in Example 1.

Combine ingredients 6 and 7 in a pharmaceutically acceptable organic solvent(s) or alkali solution and coat the tablet cores using conventional techniques.

| EXAMPLE 6 | | |
|---|---|---|
| Ingredient | mg/tablet | % (w/w) |
| 1. diltiazem HCl | 180.0 | 19.26 |
| 2. glyceryl monostearate | 311.6 | 33.33 |
| 3. microcrystalline cellulose, N.F. | 366.6 | 39.22 |
| 4. povidone USP | 42.2 | 4.51 |
| 5. magnesium stearate | 6.4 | 0.68 |
| 6. ethyl cellulose | 15.4 | 1.65 |
| 7. hydroxypropyl methyl cellulose | 7.0 | 0.75 |
| 8. color dispersion | 5.6 | 0.60 |
| Total: | 934.8 | 100.00 |

Prepare tablet cores from ingredients 1 though 5 as described in Example 1. Combine ingredients 6 through 8 and coat tablets as described in Example 5.

| EXAMPLE 7 | | |
|---|---|---|
| Ingredient | mg/tablet | % (w/w) |
| 1. diltiazem HCl | 166.0 | 17.91 |
| 2. glyceryl monostearate | 287.4 | 31.01 |
| 3. microcrystalline cellulose | 338.1 | 36.48 |
| 4. povidone USP | 38.9 | 4.20 |
| 5. magnesium stearate | 5.9 | 0.64 |
| 6. cellulose acetate phthalate | 35.2 | 3.80 |
| 7. diethyl phthalate | 8.8 | 0.95 |
| 8. diltiazem HCl | 14.0 | 1.51 |
| 9. hydroxypropyl methyl cellulose (E5) | 9.1 | 0.98 |
| 10. hydroxypropyl methyl cellulose (E50) | 6.9 | 0.74 |
| 11. polyethylene glycol (NF M.W. 3350) | 3.1 | 0.34 |
| 12. color dispersion | 13.3 | 1.44 |
| Total: | 926.7 | 100.00 |

Step A) Prepare tablet cores from ingredients 1 though 5 as described in Example 1.

Step B) Combine ingredients 6 and 7 and coat tablet cores from Step A as described in Example 5.

Step C) Combine ingredients 8 through 12 and coat tablets prepared in Step B as described in Example 5.

When the tablets are analyzed in terms of formulation stability for at least a 6 month period, the dissolution profile is virtually unchanged over an extended period of time.

While Applicant has described what is believed to be the best mode for practicing the invention, numerous alternative embodiments are contemplated as falling within the scope of the invention described herein. Consequently, the scope of the claims is not to be limited thereby.

**Claims**

1. A sustained release diltiazem tablet comprising 20 to 500 mg diltiazem or a pharmaceutically acceptable salt thereof in a hydrophobic matrix.

2. A tablet as defined in claim 1 comprising 30 to 360 mg diltiazem or a pharmaceutically acceptable salt thereof.

3. A tablet as defined in claim 1 or 2 wherein the hydrophobic matrix comprises one or more of ethylcellulose, a mixture of mono- and diglycerides, cellulose acetate, calcium phosphate, cellulose acetate butyrate and microcrystalline cellulose.

4. A tablet as defined in claim 1, 2 or 3 comprising a mixture of mono- and diglycerides in an amount of from about 20 to about 50 percent of the total tablet weight.

5. A tablet as defined in any of claims 2 to 4 further comprising microcrystalline cellulose in an amount of from about 20 to about 50 percent of the total tablet weight.

6. A tablet as defined in any of claims 1 to 5 wherein the hydrophobic matrix further comprises a binder in an amount of from about 3 to about 10 percent of the total tablet weight.

7. A tablet as defined in claim 6 wherein the binder is povidone.

8. A tablet as defined in any of claims 1 to 7 further comprising magnesium stearate in an amount of from about 0.5 to about 2 percent of the total tablet weight.

9. A tablet as defined in any of claims 1 to 8 further comprising a water-soluble or a barrier film coating wherein said coating comprises:

a) in the case of a water-soluble coating, a swelling hydrophilic polymer in an amount of from about 0.5 to about 5 percent of the total tablet weight; and in the case of a barrier coating, a water-insoluble polymer, an enteric polymer or a mixture thereof, in an amount of from about 0.5 to about 5 percent of the total tablet weight, said barrier coating optionally comprising a channelling agent selected from a hydrophilic or water-insoluble water-permeable polymer in an amount of from about 0.5 to about 2 percent;

b) a plasticizer in an amount of from about 0.1 to about 0.5 percent of the total tablet weight; and

c) optionally, a color dispersion in an amount of from about 0.5 to about 3 percent of the total tablet weight.

10. A tablet as defined in claim 9 wherein the water-soluble film coating comprises hydroxypropyl methylcellulose, polyethylene glycol 3350 and a color dispersion comprising propylene glycol, povidone and titanium dioxide.

11. A tablet as defined in claim 9 wherein the barrier coating comprises one or more of diethyl phthalate, cellulose acetate phthalate, ethyl cellulose, hydroxypropyl methylcellulose and a color dispension.

12. A tablet as defined in any of claims 1 to 11 wherein up to 25% of the diltiazem in the tablet is present in a water-soluble film coating, wherein said water-soluble coating is as defined in claim 9.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | EP-A-0 315 197 (GOEDECKE AG)<br>* whole document * | 1-3,7 | A 61 K 9/20<br>A 61 K 9/22 |
| P,A | EP-A-0 318 398 (ETHYPHARM)<br>* claims * | 1-12 | A 61 K 9/26<br>A 61 K 9/30<br>A 61 K 9/52 |
| X | WO-A-8 706 130 (LABORATOIRES DELAGRANCE)<br>* whole document * | 1-3,7,8 | |
| X | GB-A-2 163 648 (NIPPON SHINYAKU CO. LTD.)<br>* whole document * | 1-4 | |
| X | WO-A-8 808 299 (MC NEILAB INC.)<br>* whole document * | 1-3,5-7 | |
| X | BE-A- 649 386 (SMITH KLINE & FRENCH LABORATORIES)<br>* whole document * | 1-4,8 | |
| A | PATENT ABSTRACTS OF JAPAN<br>vol. 11, no. 184 (C-427)(2631), 12 June 1987; & JP - A - 62 5916 (NIPPON IYAKUHIN KOGYO K.K.) 12.01.1987 (Cat. D) | 1-12 | **TECHNICAL FIELDS SEARCHED (Int. Cl.5)**<br><br>A 61 K |

The present search report has been drawn up for all claims

| Place of search | Date of completion of the search | Examiner |
|---|---|---|
| BERLIN | 08-02-1990 | SIATOU E |